(19)

**Europäisches Patentamt**
**European Patent Office**
**Office européen des brevets**

(11) **EP 4 778 453 A1**

(12) **EUROPEAN PATENT APPLICATION**

(43) Date of publication:
   **22.07.2026   Bulletin 2026/30**

(21) Application number: **25203113.3**

(22) Date of filing: **18.09.2025**

(51) International Patent Classification (IPC):
   **A61B 5/021** (2006.01)     **A61B 5/022** (2006.01)
   **A61B 5/0225** (2006.01)

(52) Cooperative Patent Classification (CPC):
   **A61B 5/02208; A61B 5/02141; A61B 5/02233;**
   **A61B 5/0225;** A61B 2562/0247

(84) Designated Contracting States:
   **AL AT BE BG CH CY CZ DE DK EE ES FI FR GB**
   **GR HR HU IE IS IT LI LT LU LV MC ME MK MT NL**
   **NO PL PT RO RS SE SI SK SM TR**
   Designated Extension States:
   **BA**
   Designated Validation States:
   **GE KH LA MA MD TN**

(30) Priority:   **17.01.2025   IN 202521004029**

(71) Applicant: **Tata Consultancy Services Limited**
   **Mumbai, Maharashtra 400 021 (IN)**

(72) Inventors:
   • **LINGAYAT, Sushrut Suresh**
     **400601 Thane (IN)**

   • **KIMBAHUNE, Sanjay Madhukar**
     **400021 Mumbai (IN)**
   • **SHINDE, Sujit Raghunath**
     **400607 Thane (IN)**
   • **BHAVSAR, Karan Rajesh**
     **400607 Thane (IN)**
   • **VISHWAKARMA, Harsh**
     **453112 Indore (IN)**
   • **KHANDELWAL, Sundeep**
     **201301 Noida (IN)**
   • **PODUVAL, Murali**
     **400601 Mumbai (IN)**

(74) Representative: **Goddar, Heinz J.**
   **Boehmert & Boehmert**
   **Anwaltspartnerschaft mbB**
   **Pettenkoferstrasse 22**
   **80336 München (DE)**

(54) **BLOOD PRESSURE (BP) MEASUREMENT USING MAGNETO RHEOLOGICAL FLUID AND KOROTKOFF SIGNAL FEEDBACK**

(57)   Cuffs for BP measurement fail to adjust to optimal pressure to observe Korotkoff sound. A smart cuff technology comprising a MR fluid at middle layer controlled by electromagnets arranged into an outer layer of the elastic cuff is provided. MR fluid under influence of active electromagnets automatically detects the shape/curves of a subject to provide clean fit on arm of a subject. The piezoelectric sensor array senses pressure exerted on arm of the subject and electromagnets actuate MR fluid in such a way that cuff takes form of the shape of the subject. A cuff inflation control system calculates and applies enough pressure to stop blood flow so that there is no excess pressure on the upper arm and artery. The system receives feedback from microphone sensor array, attached to the sleeve, and detects the blood flow if present, not exceeding the desired pressure to stop the flow of blood.

200 — Ⓐ

210

initiating a Blood Pressure (BP) measurement cycle after detecting the pressure equivalent to the offset value in the plurality of quadrants, the BP measurement cycle comprising:

   actuating the plurality of electromagnets via the PWM circuit to inflate the cuff at a steady inflation rate by increasing the pressure at a steady rate until a blood flow in the arm stops, wherein the blood flow is sensed for a no-blood-flow-sound via a microphone array placed within the cuff (210a);

   deactivating the plurality of electromagnets to deflate the cuff by releasing the pressure at a steady deflate pressure rate and restarting the blood flow (210b);

   detecting a start Korotkoff sound and a stop Korotkoff sound of the restarted blood flow via the microphone array (210c); and

   estimating via a trained Deep Learning model a systolic BP and diastolic BP by processing a cuff pressure sensed for the start Korotkoff sound and the stop Korotkoff sound (210d)

**FIG. 2B**

EP 4 778 453 A1

**Description**

CROSS-REFERENCE TO RELATED APPLICATIONS AND PRIORITY

The present application claims priority to Indian application no. 202521004029, filed on January 17, 2025.

TECHNICAL FIELD

The embodiments herein generally relate to the field of Blood Pressure (BP) measurement devices and, more particularly, to a method and system for Blood Pressure (BP) measurement using Magneto Rheological (MR) fluid filled elastic cuff and Korotkoff signal feedback.

BACKGROUND

Blood pressure (BP) serves as a vital indicator of cardiovascular health, with systolic blood pressure (SBP) reflecting the pressure during heartbeats and diastolic blood pressure (DBP) representing the pressure between beats. Hypertension, characterized by SBP equal to or exceeding 140 mmHg and/or DBP equal to or exceeding 90 mmHg, afflicts approximately 1.28 billion individuals worldwide. Recent studies underscore the importance of regular BP monitoring in predicting cardiovascular events such as heart attacks and atrial fibrillation.

There are many invasive and non-invasive methods of BP measurements, but the most commonly used non-invasive methods are auscultation method and oscillometric method. Mercury sphygmomanometer along with stethoscope is the prime example of auscultation method in which BP is measured by inflatable cuff encircling upper arm along with a stethoscope to detect Korotkoff sounds made by the flow of blood. Measurements taken from mercury sphygmomanometer are still considered as gold standard. However, in oscillometric method, as the cuff deflates, arterial wall oscillates due to blood flow in the artery. These small wave oscillations are measured, and BP is then estimated via an empirical algorithm. Oscillometric based BP machines use the maximum volume change as an indication of the average of the systolic and diastolic BP within the artery. By combining this average with the rate of change of the pressure wave, the machines then use a variety of algorithms to estimate the systolic and diastolic BP. Oscillometric based BP devices differ with respect to their algorithms, transducers, inflation and deflation rates, cuff sizes and materials, all of which may affect the estimation of BP. Some devices may be accurate in one subject group but not necessarily in others e.g. in obese or pregnant subjects. Hence, oscillometric devices are less accurate and their BP estimation varies.

Conventional clinical auscultation method for Blood Pressure (BP) entails skilled observers using stethoscopes to listen for Korotkoff sounds and estimate systolic and diastolic BP values of a subject or patient being monitored. Auscultation is a method used to listen to the sounds of the body during a physical examination by using a stethoscope. To measure BP, doctors listen to the Korotkoff sound and observe the cuff pressure values on sphygmomanometer. Cuff pressure value at the start of Korotkoff sound is known as systolic blood pressure (SBP), and cuff pressure value at the end of the Korotkoff sound is known as diastolic blood pressure (DBP). This is considered as the gold standard method for BP measurement and all the measurements are verified and validated against this method. Using this method, BP measurement is heavily dependent upon the expertise and physical condition of the user and there may be human error while measurement. If the user monitoring the has bad hearing ability or bad vision, it will lead to variable measurements. Also, in cuff-based BP measurement, there is a long-term issue of artery damage. Repeatedly inflating cuff excessively and measuring BP can cause artery damage and current devices or methods do not take account for the damage. Thus, current cuff designs have limitations to provide best or optimal fitting the arm leading to over pressure scenarios that damage the artery in long term. Further, the unwanted noise presence that is inherently present along with Korotkoff sound further adds to wrong recording of BP values in existing automated BP estimation methods.

Some existing methods use flexible fillers so that they enable the cuff to take shape of arm of the subject. The existing mechanisms use air as filler to inflate the cuff, but the rate of change of pressure cannot be controlled precisely. Due to this, the cuff may get inflated with excess pressure which causes artery damage. Also, existing mechanisms do not track the blood flow in the artery as feedback to inflation mechanism, which may lead to excess cuff pressure and artery damage. In some cases, the cuff apparatus has multiple cushion bags in inflated state which provides better coverage, no wrinkles. But in this scenario, the exact shape of the arm is not considered, and this mechanism is only focused on cuff inflation in less power and short time.

SUMMARY

Embodiments of the present disclosure present technological improvements as solutions to one or more of the above-mentioned technical problems recognized by the inventors in conventional systems.

For example, in one embodiment, a method for Blood Pressure (BP) measurement.is provided. The method includes computing an initial pressure exerted across each of a plurality of quadrants of an elastic cuff fitted on an arm of a subject, wherein the initial pressure exerted per quadrant is sensed by an inner layer of the elastic cuff comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid.

Further the method includes computing an average pressure per quadrant by averaging the initial pressure sensed by each of a piezoelectric sensor among the plurality of sensors in associated quadrant.

Further the method includes identifying a quadrant exerting a highest average pressure on the arm from among the plurality of quadrants, wherein the highest pressure is identified as an offset pressure value.

Furthermore the method includes actuating via a Pulse Width Modulation (PWM) circuit, the plurality of electromagnets in remaining quadrants other than the quadrant of the highest average pressure to inflate the elastic cuff to create a pressure equivalent to the offset value in the plurality of quadrants enabling a clean fitting of the elastic cuff around the arm due to a viscosity change of the MR fluid under pressure.

Further the method includes initiating a Blood Pressure (BP) measurement cycle after detecting the pressure equivalent to the offset value in the plurality of quadrants, the BP measurement cycle comprising: actuating the plurality of electromagnets via the PWM circuit to inflate the elastic cuff at a steady inflation rate by increasing the pressure at a steady rate until a blood flow in the arm stops, wherein the blood flow is sensed for a no-blood-flow-sound via a microphone array placed within the elastic cuff; deactivating the plurality of electromagnets to deflate the elastic cuff by releasing the pressure at a steady deflate pressure rate and restarting the blood flow; detecting a start Korotkoff sound and a stop Korotkoff sound of the restarted blood flow via the microphone array; and estimating via a trained Deep Learning model a systolic BP and diastolic BP by processing a cuff pressure sensed for the start Korotkoff sound and the stop Korotkoff sound.

In another aspect, a system for BP measurement is provided. The system comprises an elastic cuff providing a sleeve fitting around an arm of a subject. The elastic cuff comprises an inner layer having an arrangement of the plurality of piezo electric sensors spaced evenly into a plurality of quadrants, and a middle layer filled with a MR fluid, and an outer layer having an arrangement of a plurality of electromagnets spaced evenly into the plurality of quadrants; a triangular microphone array placed above the outer layer, and a cuff pressure sensor to sense cuff pressure. The controller comprising a memory storing instructions; one or more Input/Output (I/O) interfaces; and one or more hardware processors coupled to the memory via the one or more I/O interfaces, wherein the one or more hardware processors are configured by the instructions to compute an initial pressure exerted across each of a plurality of quadrants of an elastic cuff fitted on an arm of a subject, wherein the initial pressure exerted per quadrant is sensed by an inner layer of the elastic cuff comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid.

Further the controller is configured to compute an average pressure per quadrant by averaging the initial pressure sensed by each of a piezoelectric sensor among the plurality of sensors in associated quadrant.

Further the controller is configured to identify a quadrant exerting a highest average pressure on the arm from among the plurality of quadrants, wherein the highest pressure is identified as an offset pressure value.

Furthermore the controller is configured to actuate, via a Pulse Width Modulation (PWM) circuit, the plurality of electromagnets in remaining quadrants other than the quadrant of the highest average pressure to inflate the elastic cuff to create a pressure equivalent to the offset value in the plurality of quadrants enabling a clean fitting of the elastic cuff around the arm due to a viscosity change of the MR fluid under pressure.

Further the controller is configured to initiate a Blood Pressure (BP) measurement cycle after detecting the pressure equivalent to the offset value in the plurality of quadrants, the BP measurement cycle comprising: actuating the plurality of electromagnets via the PWM circuit to inflate the elastic cuff at a steady inflation rate by increasing the pressure at a steady rate until a blood flow in the arm stops, wherein the blood flow is sensed for a no-blood-flow-sound via a microphone array placed within the elastic cuff; deactivating the plurality of electromagnets to deflate the elastic cuff by releasing the pressure at a steady deflate pressure rate and restarting the blood flow; detecting a start Korotkoff sound and a stop Korotkoff sound of the restarted blood flow via the microphone array; and estimating via a trained Deep Learning model a systolic BP and diastolic BP by processing the pressure sensed for the start Korotkoff sound and the stop Korotkoff sound.

In yet another aspect, there are provided one or more non-transitory machine-readable information storage mediums comprising one or more instructions, which when executed by one or more hardware processors causes a method for Blood Pressure (BP) measurement.is provided. The method includes computing an initial pressure exerted across each of a plurality of quadrants of an elastic cuff fitted on an arm of a subject, wherein the initial pressure exerted per quadrant is sensed by an inner layer of the elastic cuff comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid.

Further the method includes computing an average pressure per quadrant by averaging the initial pressure sensed by each of a piezoelectric sensor among the plurality of sensors in associated quadrant.

Further the method includes identifying a quadrant exerting a highest average pressure on the arm from among the plurality of quadrants, wherein the highest pressure is identified as an offset pressure value.

Furthermore the method includes actuating via a Pulse Width Modulation (PWM) circuit, the plurality of electromagnets in remaining quadrants other than the quadrant of the highest average pressure to inflate the elastic cuff 114 to create a pressure equivalent to the offset value in the plurality of quadrants enabling a clean fitting of the elastic cuff around the arm due to a viscosity change of the MR fluid under pressure.

Further the method includes initiating a Blood Pressure (BP) measurement cycle after detecting the pressure equivalent to the offset value in the plurality of quadrants, the BP measurement cycle comprising: actuating the plurality of electromagnets via the PWM circuit to inflate the elastic cuff at a steady inflation rate by increasing the pressure at a steady rate until a blood flow in the arm stops, wherein the blood flow is sensed for a no-blood-flow-sound via a microphone array placed within the elastic cuff; deactivating the plurality of electromagnets to deflate the elastic cuff by releasing the pressure at a steady deflate pressure rate and restarting the blood flow; detecting a start Korotkoff sound and a stop Korotkoff sound of the restarted blood flow via the microphone array; and estimating via a trained Deep Learning model a systolic BP and diastolic BP by processing a cuff pressure sensed at the start Korotkoff sound and the stop Korotkoff sound.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of a system for Blood Pressure (BP) measurement using Magneto Rheological (MR) fluid filled elastic cuff and Korotkoff signal feedback, in accordance with some embodiments of the present disclosure.

FIG. 1B illustrates an architectural overview of the system of FIG. 1A, in accordance with some embodiments of the present disclosure.

FIGS. 2A and 2B is a flow diagram illustrating a method for BP measurement using MR fluid filled elastic cuff and Korotkoff signal feedback, using the system depicted in FIG. 1A and 1B, in accordance with some embodiments of the present disclosure.

FIG. 3 and FIG. 4 depict the layered structure of the elastic cuff, in accordance with some embodiments of the present disclosure.

FIGS. 5A and 5B depict functioning of the elastic cuff, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Embodiments of the present disclosure provide a method and system for Blood Pressure (BP) measurement using Magneto Rheological (MR) fluid filled elastic cuff and Korotkoff signal feedback. The method and system herein discloses a digital auscultatory based BP measurement method with personalized smart cuff inflation technology using MR fluid. This method follows the conventional gold standard method of auscultation, but the Korotkoff sound are observed by a triangular microphone sensor array and the cuff pressure is observed via a plurality of piezoelectric sensors, eliminating subjectivity and recording errors due to human involvement. The piezo electric sensors are arranged in quadrant-based circular structure in the innermost layer of the elastic cuff, interchangeably referred to as cuff hereinafter.

The cuff is a smart cuff worn like a sleeve and comprises a MR fluid at the middle layer, controlled by electromagnets arranged into quadrants at the outer layer. The MR fluid, which changes viscosity under the magnetic field if put under influence of active electromagnets to automatically spread and fit taking shape of the curves of an arm of the subject to provide clean or optimal fit when worn like a sleeve. The piezoelectric sensor array gives feedback of the pressure exerted on an arm or limb of the subject when the electromagnets actuate so as to enable control pressure to the right level to just cause 'stop flow' point of the blood through the artery. This provides optimal needed pressure while comforting the subject without unnecessary additional pressure. A microphone sensor array, attached to outermost layer of the cuff detects the blood flow and accordingly the pressure activation is continued or terminated. This allows the system to precisely control the cuff pressure and not to exceed the desired pressure to stop the flow of blood.

The system also enables a cuff pressure personalization system which takes account of how much cuff pressure and rate of cuff pressure should be applied according to the age, gender and BMI of the patient.

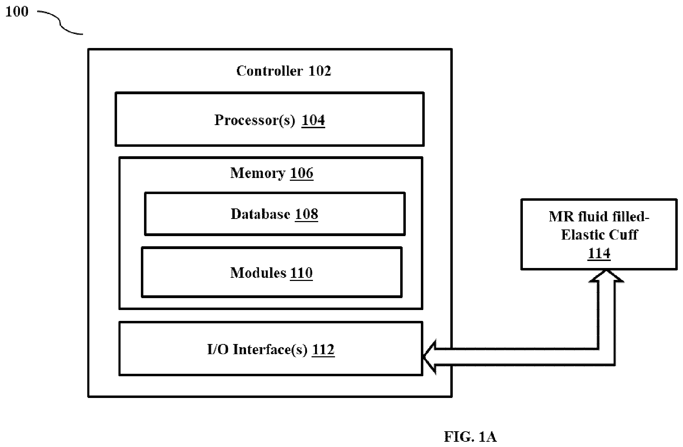

Referring now to the drawings, and more particularly to FIGS. 1A through 5B, where similar reference characters denote corresponding features consistently throughout the figures, there are shown preferred embodiments, and these embodiments are described in the context of the following exemplary system and/or method.

FIG. 1A is a functional block diagram of a system 100 for Blood Pressure (BP) measurement using Magneto Rheological (MR) fluid filled elastic cuff, in accordance with some embodiments of the present disclosure. The system comprises a controller 102 for BP measurement, which controls actuation of the elastic cuff 114 in accordance with a BP measurement cycle.

In an embodiment, the controller 102 includes a processor(s) 104, communication interface device(s), alternatively referred as input/output (I/O) interface(s) 112, and one or more data storage devices or a memory 106 operatively coupled to the processor(s) 104. The controller 102 with one or more hardware processors is configured to execute functions of one or more functional blocks of the controller 102.

Referring to the components of controller 102, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 106. In one embodiment, the controller 102 can be implemented in a variety of computing systems including laptop computers, notebooks, hand-held devices such as mobile phones, workstations, mainframe computers, servers, and the like. In another embodiment, the controller can be implemented as system on chip (SoC), where all the processing and communication will be on the board. In another embodiment, the controller can be implemented as a cloud interface, in which WiFi or GSM module takes care of the communication between sensors of the elastic cuff 114 and controller 112.

The I/O interface(s) 106 can include a variety of software and hardware interfaces, for example, a web interface, a graphical user interface and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices.

The memory 106 may include any computer-readable medium known in the art including, for example, volatile memory, such as static random access memory (SRAM) and dynamic random access memory (DRAM), and/or nonvolatile memory, such as read only memory (ROM), erasable programmable ROM, flash memories, hard disks, optical disks, and magnetic tapes.

In an embodiment, the memory 106 includes a plurality of modules 110 such as a cuff inflation control system and a cuff deflation control system and the like.

The plurality of modules 110 include programs or coded instructions that supplement applications or functions performed by the system 100 for executing different steps involved in the process of BP measurement being performed by the system 100. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

Further, the memory 106 may comprise information pertaining to input(s)/output(s) of each step performed by the processor(s) 104 of the system100 and methods of the present disclosure.

Further, the memory 106 includes a database 108. The database (or repository) 108 may include a plurality of abstracted pieces of code for refinement and data that is processed, received, or generated as a result of the execution of the plurality of modules in the module(s) 110.

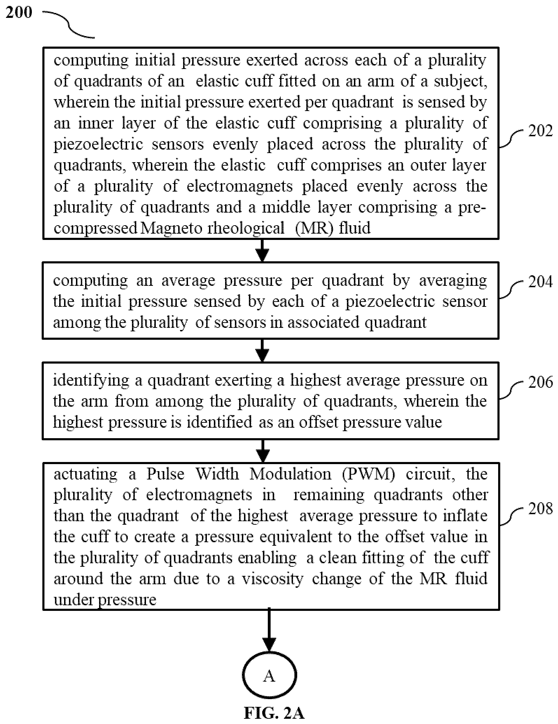

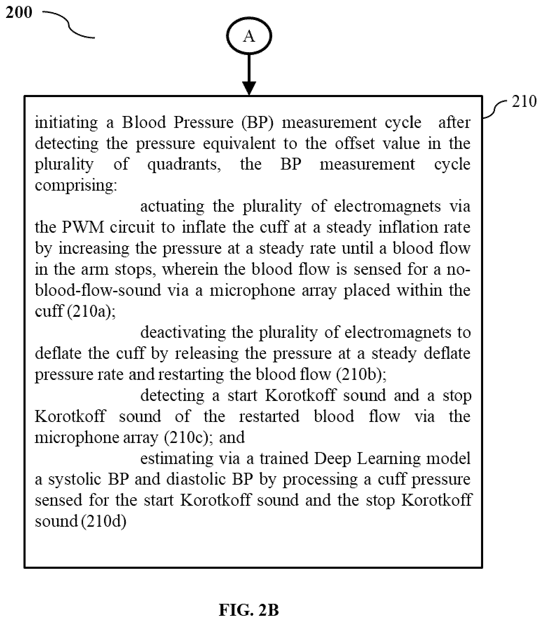

Although the database 108 is shown internally to the controller 102, it will be noted that, in alternate embodiments, the database 108 can also be implemented external to the controller 102 and communicatively coupled to the controller 102. The data contained within such external database may be periodically updated. For example, new data may be added into the database (not shown in FIG. 1A) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the controller 102 are now explained with reference to steps in flow diagrams in FIG. 2 through FIG. 5B.

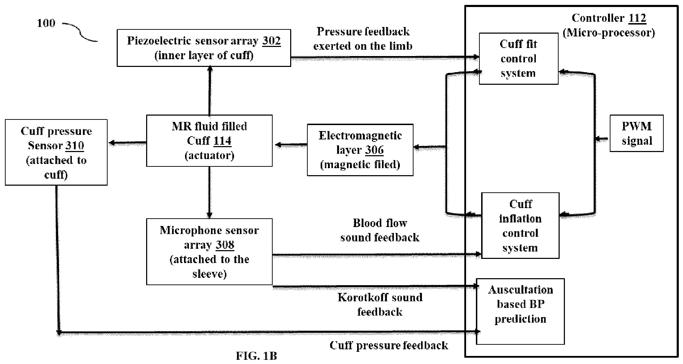

FIG. 1B illustrates an architectural overview of the system of FIG. 1A, in accordance with embodiments of the disclosure. Two main sub parts of the system 100 are the controller 102 and the elastic cuff 114. The elastic cuff 114 is a compression sleeve which consists of pre-compressed MR fluid 304 filled cuff, cuff pressure sensor (piezoelectric sensors) and microphone sensor array 308. The other part, the controller 102, consists of a powerful microprocessor which contains a plurality of modules 110 such as the cuff inflation control system, the cuff deflation control system, and auscultation-based BP prediction algorithm. These two parts are connected to each other via sensor's feedback.

The microprocessor (one or more hardware processors 104) actuates the electromagnet (306), which in turn changes the viscosity of the MR fluid, which causes pressure on the upper arm. This pressure is sensed by piezoelectric sensor array, which is in the inner layer of compression sleeve. This pressure feedback is fed to cuff inflation control system. Also, microphone sensor array, which is also attached to the sleeve, first gives blood flow sound feedback at cuff inflation, and then gives Korotkoff sound feedback at cuff deflation.

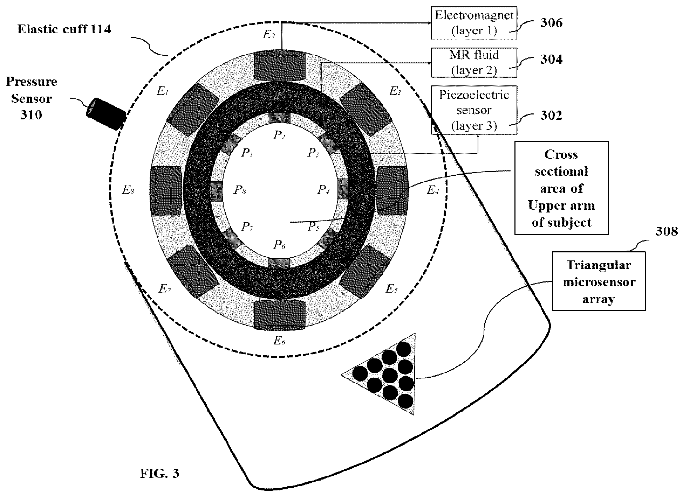

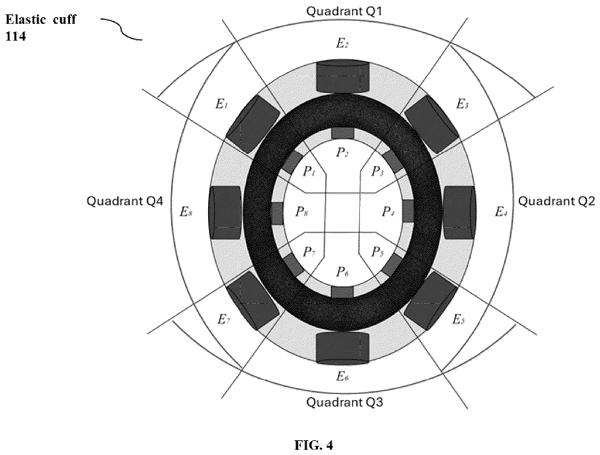

**MR fluid filled elastic cuff-** FIG. 3 and FIG. 4 depict the layered structure of the elastic cuff 114 with a sleeve fitting, in accordance with some embodiments of the present disclosure. The sleeve fitting herein refers to a compression sleeve consisting of the elastic cuff 114 filled with MR fluid and sealed with a pressure sensor to monitor the cuff pressure for BP measurement. There are 4 quadrants designated (Q1, Q2, Q3, Q4) which comprise of 3 sets of piezoelectric sensor and electromagnets. The MR fluid filled cuff is the actual actuator which plays an important role in controlling cuff pressure. FIG. 3 show the cuff design and layers incorporated in the sleeve fitting. The outermost layer consists of electromagnets (306), which are distributed evenly, and are divided into 4 quadrants, which are Upper limb or arm, Right side limb or arm, Lower limb or arm and Left side limb or arm.

    Quadrant Q1 => P1, P2, P3 (piezoelectric sensors) and E1, E2, E3 (electromagnets)
    Quadrant Q2 => P2, P3, P4 (piezoelectric sensors) and E2, E3, E4 (electromagnets)
    Quadrant Q3 => P4, P5, P6 (piezoelectric sensors) and E4, E5, E6 (electromagnets)
    Quadrant Q4 => P6, P7, P1 (piezoelectric sensors) and E6, E7, E1 (electromagnets)

The middle layer contains MR fluid (304) which is in liquid state under normal circumstances, and the innermost layer contains piezoelectric sensor arrays (302), which are situated under every electromagnet (306), to give pressure feedback. The sleeve can easily be put on the upper arm and when the magnetic field is induced into the inward direction, the MR fluid becomes semi solid, which then tightens the cuff and applies pressure onto the upper arm and stops the blood flow inside the artery. To not damage the artery by applying excess pressure, a control action via the controller 102 is implied to continuously monitor the applied pressure on the arm, and the pressure inside the cuff.

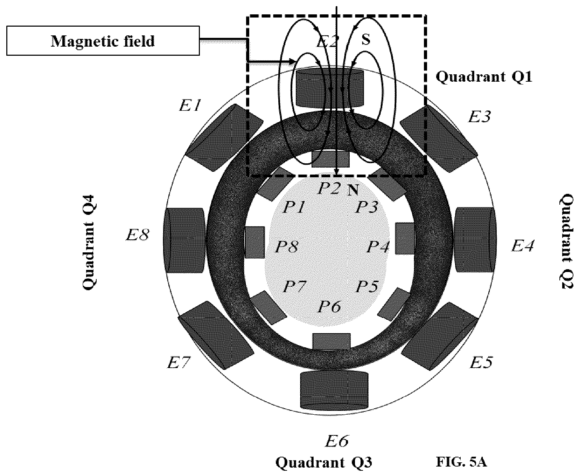

As can be seen in FIG. 5A, the magnetic field strength (B) at the center of the circular coil carrying current $I$ with n number of turns is calculated by:

$$ B = \frac{\mu_0 n I}{2r} \tag{1} $$

where $\mu_0$ = magnetic permeability of free space and r = radius of coil.

- Electromagnets in Q1, Q2, and Q3 induces magnetic field which changes the viscosity of MR fluid.
- The magnetic field shown in FIG. 5A is induced by all the electromagnets in Q1, Q2 and Q3, i.e. E1, E2, E3, E4, E5, E6, E7.

The magnetic field intensity is calculated by the magnetic field induction at a point on the axis of a circular coil carrying current is calculated by:

$$ B = \frac{\mu_0 n I r^2}{2(r^2+x^2)^{\frac{3}{2}}} \tag{2} $$

Where r = radius of coil, $xI$ = distance from center of the circular coil, $I$= current, $n$= no of turns. If simply it is assumed that $x = 0$, then the above equation 1 is obtained. Here, $xI$ is also constant since the MR fluid and electromagnets are stationary and not moving. Also, the r and $nl$ are constant in the current use case, so magnetic field strength is directly proportional to the current. By increasing the current, the strength of magnetic field can be increased. Now, if the I is replaced with an Integrated Pulse Width Modulation (IPWM) which is the current input after PWM is implemented, then IPWM = PWM × I

If the PWM duty cycle is 10% and input current is 10 Ampere (Amp.), then IPWM will be 1 Amp. So, magnetic field strength will be,

$$B = \frac{\mu_0 n I_{PWM} r^2}{2(r^2+x^2)^{\frac{3}{2}}} \qquad (3)$$

By controlling the current in the coil, the magnetic field strength of every electromagnet can be controlled. Just like the average pressure value, the average magnetic field strength can be evaluated for every quadrant. This average magnetic field strength is then correlated with the viscosity of MR fluid under its influence. In the absence of a magnetic field, they behave like a Newtonian fluid with a low viscosity of 0.1 to 1.0 Pa·s (Pascal second). The correlation between magnetic field strength and change in viscosity is derived from this experiment. From this, it is observed that as soon as magnetic induction crosses a certain threshold, the viscosity of MR fluid rises exponentially.

In the elastic cuff 114, the MR fluid by design is already compressed, and in studies show that the yield stress of MR fluids after compression is much stronger than that of uncompressed MR fluids under the same applied current. So, for example, the SG MRF2035 (MR fluid) without compression process has a yield stress of about 53kPa. But when the current of 2.5A is applied inducing magnetic field and the compressive stress of 2.0MPa is also applied, the yield stress exceeds 1100kPa. This experiment shows that it is possible to attain high yield stress with MR fluid.

Now to calculate the amount of pressure/stress exerted by MR fluid on the limb, a relation is derived from the above experiment, which is, $\sigma = \frac{F}{S}$, where $F$ is the force applied by the outer layer of the cuff and S is the surface area of the cuff. So, if it is considered that the electromagnet $E_1$ is inducing magnetic field $B_1$ onto the MR fluid situated at distance $x_1$ from the coil, which causes change in the viscosity of MR fluid. But since MR fluid is in compression state, the force exerted by the outer layer of cuff will be $F_1$. Now if the surface area under electromagnet $E_1$ is $S_1$, the pressure exerted by the MR fluid in area $S_1$ will be $\sigma_1$. In this way, the pressure exerted by the MR fluid on the upper limb can be calculated. To get the desired pressure, first the composition of the MR fluid needs to be considered. Then the maximum yield stress can be calculated for that composition of MR fluid. After that, the pressure can be directly controlled by changing the PWM input, which changes the current in the coil. The pressure on the arm is captured by piezoelectric sensors from respective quadrants. When the average pressure of all quadrants is equal, the system halts magnetic induction, and the cuff is fitted properly. The pressure exerted by $E_1$ is calculated by $\sigma = \frac{F}{S}$.

**Piezoelectric sensor array (302)-** As shown FIG. 4 the piezoelectric sensor array is situated at the innermost layer. The piezoelectric material is a quartz crystal which dissipates small voltages when under stress or pressure. This voltage can be captured by applied sensor and stress can be converted into voltage value, where applied stress is directly proportional to the voltage generated. The main use case of it is automatic shape detection by giving pressure feedback to the cuff fit control system at each point. This layer and the number of piezoelectric sensors is spread across the sleeve and is subject to change according to the design and size of the sleeve. Commercially available piezoelectric transducer generates voltages in the range of 10mV to 100mV. To detect these voltages, an amplifier is required to convert these signals into detectable voltages (3.3V to 5V). The correlation between applied pressure and output voltage is linear, i.e., directly proportional.

**Microphone sensor array (308)-** The microphone sensor array (as depicted in FIG. 3 consists of electret microphones, which convert vibrations/sound waves into electrical signals. The major drawback of the auscultation method is to locate the brachial artery and to put the bell of stethoscope on the located point. To address this problem, a triangular shaped microphone sensor array is designed, which is attached to the cuff in such a way that it comes up on top of the cubital fossa of the arm when the sleeve fit cuff is worn by the subject. This location is where the brachial artery is located and since the pulse and blood flow sound is very low in amplitude, vibrations captured by multiple microphone sensors amplifies the net sound resulting in clear detection of blood flow sound and Korotkoff sound. Also, in general BP devices, motors are used to inflate the cuff, which produces lot of vibrations, making it impossible to detect blood flow sound. But in the system herein, since MR fluid does not make any kind of sound, it is very possible to detect the blood flow in real time.

The major use cases of microphone sensor array are while inflation, detection of the blood flow sound, and while deflation, detection of Korotkoff sound. This microphone sensor array plays a critical role in both cuff inflation and cuff deflation.

**Cuff pressure sensor (310)-** To measure the BP, the crucial part is the cuff pressure at start and stop of the Korotkoff sound. To measure cuff pressure throughout inflation and deflation, the cuff pressure sensor 310 (also referred to a pressure sensor 310) as depicted in FIG.3 is attached to the MR fluid cuff, which measures the pressure inside the cuff. This pressure feedback is then used as an input for BP prediction. Deep learning model needs both Korotkoff sound and cuff pressure. For DL model training and prediction, the cuff pressure can be obtained from only the cuff pressure sensor 310 since the piezo electric sensors 302 cannot provide or measure cuff pressure.

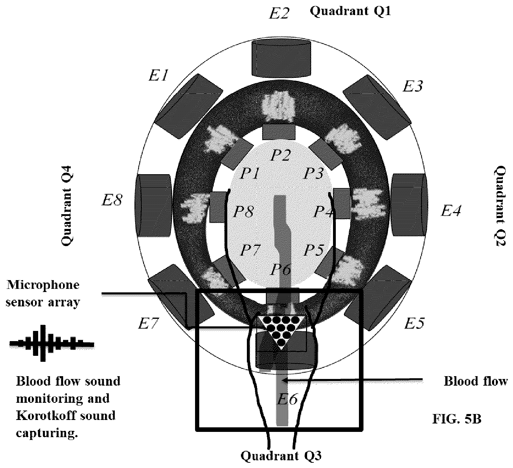

**Cuff fit control system-** As depicted in FIG. 5B, a transfer function is a mathematical function that models the system's output for each possible input. The cuff fit control system works on the following transfer function-

$$TF_{fit} = \frac{G_{PWM}}{(1 \pm G_{PWM} \cdot H_{pressure})} \qquad (4)$$

Where GPWM is the open loop gain of PWM input. This sets the rate of change of PWM value, which in turn changes the current input. $H_{pressure}$ is the feedback gain which depends on the error value in pressure feedback.

**Cuff inflation control system-** The cuff inflation control system works on the following transfer function-

$$TF_{inflate} = \frac{G_{PWM}}{(1 \pm G_{PWM} \cdot H_{sound})} \qquad (5)$$

Where GPWM is the open loop gain of PWM input. This sets the rate of change of PWM value, which in turn changes the current input. $H_{sound}$ is the feedback gain which depends on the error value in sound feedback. All electromagnets are activated to increase cuff pressure. The triangular microphone sensor array 308 monitors blood flow sound and gives feedback to controller 112. The cuff inflation will continue till the blood flow is completely stopped. When no blood flow sound is detected, the controller 102 halts electromagnetic induction.

**Auscultation prediction-** The traditional auscultation method of BP prediction is to observe Korotkoff sound with the help of stethoscope and observe cuff pressure values at start and stop of Korotkoff sound using sphygmomanometer. In the proposed system 100, the same principle is followed, but the observation part is done by sensors, and the calculation part is handled by machine learning algorithm. When cuff deflation starts, microphone sensor array captures Korotkoff sound and pressure sensor captures cuff pressure throughout the cuff deflation. Both the data are captured at same time, and these data are given as input to a deep learning CNN model, which predicts the timestamps as to at what time Korotkoff sound started and stopped. These timestamps values are correlated with the cuff pressure values, and the systolic and diastolic BP values are calculated. This can be done in accordance with study explained in Beevers, G., Lip, G.Y., O'Brien, E.: Blood pressure measurement: Part ii-conventional sphygmomanometry: Technique of auscultatory blood pressure measurement. Bmj 322(7293), 1043-1047 (2001) Pan, F., He, P., Chen, F., Zhang, J., Wang, H., Zheng, D.: A novel deep learning based automatic auscultatory method to measure blood pressure. International journal of medical informatics 128, 71-78 (2019)

As depicted in FIG. 5B, when the cuff starts to deflate slowly, and blood starts to flow through artery. This phenomenon produces vibrations which is Korotkoff sound. This Korotkoff sound is then captured by triangular microphone sensor array 308. Also, at the same time, pressure sensor 310 attached to the elastic cuff 114 (or cuff 114) also starts to capture pressure inside cuff 114. Now, the cuff 114 starts to deflate slowly, and blood starts to flow through artery. This phenomenon produces vibrations which is Korotkoff sound. This Korotkoff sound is then captured by triangular microphone sensor array 308 or also referred to as microphone array 308. Also, at the same time, pressure sensor attached to the cuff also starts to capture pressure inside cuff.

Auscultation is a method where blood pressure is derived with the help of cuff pressure and Korotkoff sound. The cuff pressure at the time of start of Korotkoff sound is known as systolic blood pressure and the cuff pressure at the time of end of Korotkoff sound is known as diastolic blood pressure. So, to derive blood pressure, both the data is captured at the same instance and stored with timestamps. This data is then given as input to deep learning model (such as pretrained CNN), which predicts Korotkoff peaks. Using these peaks, the controller 102 calculates BP values. After the Korotkoff sound has ended and blood is flowing normally, the cuff is deflated completely. All electromagnets are demagnetized, and MR fluid came back to its natural liquid state.

**Cuff personalization with respect to age, gender, BMI:** The rate of cuff inflation and the rate of change of pressure while measuring BP is completely dependent on a person's anatomy, which comprises age, gender and BMI. If a person is old and has a low BMI, then the pressure rate should be low, so as to not inflict pain. In the proposed system, before BP measurement, a person's age, gender and BMI to the fed to the system 100 via the UI of the I/O interface 112, and the controlled 102 then maps these values to the respective rate of change of pressure. This makes it possible to personalize cuff pressure for every user and makes the BP measurement process very comfortable.

**Working of the architecture of the system 100 depicted in FIG. 1B:** Once the system 100 is ready and set up, the user (the subject) must put on the elastic cuff 114 like a sleeve. The system 100 can then be activated manually to initiate BP measurement cycle. As soon as system 100 is activated, the piezoelectric sensors measure the initial pressure and for each quadrant average pressure exerted on the limb or arm is calculated as below:

$$Pavg = \frac{P1 + P2 + P3}{3} \qquad (6)$$

Then, whichever quadrant has the highest average pressure, it is taken as desired offset value, which other 3 quadrants will try to attain. The electromagnet induces magnetic fields which change the viscosity of MR fluid in the range of respective fields. The piezoelectric sensor situated below every electromagnet constantly gives pressure feedback to the cuff fit control system. This control system calculates average quadrant pressure, and it controls the PWM input till all the average quadrant pressure values are equal. The Pulse Width modulation (PWM) is a technique which generates a digital signal (consisting of zeros and ones). It basically turns on and off at very high frequency, to control the output voltage, in this instance, electromagnets. The percentage of PWM is the duty cycle. This helps in controlling the magnetic field strength, which in turn controls the viscosity of the MR fluid. For example, the supply current is 12 Amp, and PWM duty cycle is 50%, then the output current will be 6 Amp.

After all the quadrants are at equal pressure values, those PWM input values are set as offsets for every quadrant, to maintain the constant pressure. At this stage, cuff has taken the shape of upper arm of the user, and after that system will start inflating the cuff, by actuating all electromagnets from their respective offset PWM values onwards. At the same time, microphone sensor array observes blood flow sound and gives it as feedback to the cuff inflation control system. The cuff is inflated till no blood flow sound is detected. This is the ideal case for cuff inflation where enough pressure is exerted to stop the blood flow in artery, but not too much pressure as to damage the artery.

After that all the electromagnets maintain the pressure, and then cuff pressure sensor starts capturing the cuff pressure and microphone sensor array starts capturing Korotkoff sound. Now, cuff starts to deflate slowly and steadily, while Korotkoff sound and cuff pressure values are captured by the system. Once the cuff is deflated completely, captured data is given as input to the deep learning CNN model, which predicts systolic and diastolic blood pressure values by corelating start and stop of Korotkoff sound with cuff pressure values.

FIGS. 2A and 2B is a flow diagram illustrating the method 200 for BP measurement using MR fluid filled elastic cuff, using the system depicted in FIG. 1A and 1B, in accordance with some embodiments of the present disclosure.

In an embodiment, the controller 102 of the system 100 comprises one or more data storage devices or the memory 106 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the controller 102 as depicted in FIG. 1A and 1B and the steps of flow diagram as depicted in FIGS. 2A, and 2B. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

Referring to the steps of the method 200, at step 202 of the method 200, the one or more hardware processors 104 are configured by the instructions to compute an initial pressure exerted across each of a plurality of quadrants of an elastic cuff 114 fitted on an arm of a subject, wherein the initial pressure exerted per quadrant is sensed by an inner layer of the elastic cuff 114 comprising a plurality of piezoelectric sensors evenly placed across the plurality of quadrants. As described in FIG 3 and FIG. 4 the elastic cuff 114 comprises an outer layer of a plurality of electromagnets placed evenly across the plurality of quadrants and a middle layer comprising a pre-compressed Magneto rheological (MR) fluid.

At step 204 of the method 200, the one or more hardware processors 104 are configured by the instructions to compute an average pressure ( as in equation 6) per quadrant by averaging the initial pressure sensed by each of a piezoelectric sensor among the plurality of sensors in associated quadrant.

At step 206 of the method 200, the one or more hardware processors 104 are configured by the instructions to identifying, a quadrant exerting a highest average pressure on the arm from among the plurality of quadrants, wherein the highest pressure is identified as an offset pressure value.

At step 208 of the method 200, the one or more hardware processors 104 are configured by the instructions to actuating, via a Pulse Width Modulation (PWM) circuit, the plurality of electromagnets in remaining quadrants other than the quadrant of the highest average pressure to inflate the cuff to create a pressure equivalent to the offset value in the plurality of quadrants enabling a clean fitting of the cuff around the arm due to a viscosity change of the MR fluid under pressure.

At step 210 of the method 200, the one or more hardware processors 104 are configured by the instructions to initiate a Blood Pressure (BP) measurement cycle after detecting the pressure equivalent to the offset value in the plurality of quadrants. The BP measurement cycle is as explained with reference to working of the system 100 in FIG. 1B.

The steps of BP measurement cycle reiterated in FIG. 2 herein are as below:

1. Initially, at step 210a, actuating the plurality of electromagnets via the PWM circuit to inflate the cuff at a steady inflation rate by increasing the pressure at a steady rate until a blood flow in the arm stops. The blood flow is sensed for a no-blood-flow-sound via a microphone array placed within the cuff.

2. At the next step 210b, deactivating the plurality of electromagnets to deflate the cuff by releasing the pressure at a steady deflate pressure rate and restarting the blood flow.

3. At the next step 210c, detecting a start Korotkoff sound and a stop Korotkoff sound of the restarted blood flow via the microphone array.

4. Finally, at the last step 210d, estimating via a trained Deep Learning model (DL CNN model or simply CNN model) as explained above) a systolic BP and diastolic BP by processing a cuff pressure sensed at the start Korotkoff sound and the stop Korotkoff sound via a cuff pressure sensor.

As mentioned, the steady inflates pressure rate, and the steady deflate pressure rate is personalized for the subject by precomputing based on acquired profile information of the subject comprising gender, age and Body Mass Index (BMI).

Unlike air and liquid used by methods in the art as filler in the cuff for better fitment to the arm, the method and system disclosed herein utilizes MR fluid to fill the elastic cuff, which has a unique property to change the viscosity under magnetic field. With the help of piezoelectric sensors (used as pressure feedback) and cuff fit control system, the MR fluid filled cuff will take the shape of user's upper arm automatically.

Main advantage of using MR fluid as disclosed herein is it's high precision control. Using PWM signal, it is possible to control and change the current flow with high accuracy. This enables the magnetic field as well as viscosity of MR fluid with precise control. MR materials take shape of the arm hence inflation is more natural and less uncomfortable. Furthermore, MR fluids are typically nontoxic and do not pose any danger to human health unlike mercury

The cuff inflation and deflation technology disclosed herein uses the property that the viscosity of MR fluid changes under magnetic field. With the technology disclosed herein there is almost no noise while cuff inflation (since there is no electric motor), hence blood flow sound can be detected in real time, which is a crucial feature in BP measurement. This helps in stopping the cuff inflation at exact point, preventing damage to artery. The high precise control over viscosity of MR fluid gives high precision of rate of change of cuff pressure. MR fluid has high shear stress in compressed state, which is required to create enough pressure to stop blood flow.

The system also utilizes the blood flow sound feedback based accurate cuff inflation. Use of electromagnets and MR fluid as an actuator makes almost no noise. This makes it possible to observe blood flow while the cuff is inflated. This feedback is crucial in cuff inflation for accurate stop point inflation to avoid over pressure and damage the arteries.

The cuff pressure personalization, specifically rate of pressure increase or pressure decrease, which is controlled by the electromagnet and MR fluid mechanisms with respect to age, gender, BMI before BP measurement, makes it possible to personalize the cuff pressure for every user, and ensures medically appropriate rate of pressure change to enable rate of blood flow b withing medically tolerable limits for the user.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200) for Blood Pressure (BP) measurement, the method comprising:

    computing (202), via one or more hardware processors, an initial pressure exerted across each of a plurality of quadrants of an elastic cuff fitted on an arm of a subject, wherein the initial pressure exerted per quadrant is sensed by an inner layer of the elastic cuff comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid;
    computing (204), via the one or more hardware processors, an average pressure per quadrant by averaging the initial pressure sensed by each of a piezoelectric sensor among the plurality of sensors in associated quadrant;
    identifying (206), via the one or more hardware processors, a quadrant exerting a highest average pressure on the arm from among the plurality of quadrants, wherein the highest pressure is identified as an offset pressure value;
    actuating (208), via a Pulse Width Modulation (PWM) circuit controlled by the one or more hardware processors, the plurality of electromagnets in remaining quadrants other than the quadrant of the highest average pressure to inflate the elastic cuff to create a pressure equivalent to the offset value in the plurality of quadrants enabling a clean fitting of the elastic cuff around the arm due to a viscosity change of the MR fluid under pressure; and
    initiating (210), via the one or more hardware processors, a Blood Pressure (BP) measurement cycle after detecting the pressure equivalent to the offset value in the plurality of quadrants, the BP measurement cycle comprising:

        actuating the plurality of electromagnets via the PWM circuit to inflate the elastic cuff at a steady inflation rate by increasing the pressure at a steady rate until a blood flow in the arm stops, wherein the blood flow is sensed for a no-blood-flow-sound via a microphone array placed within the elastic cuff (210a);
        deactivating the plurality of electromagnets to deflate the elastic cuff by releasing the pressure at a steady deflate pressure rate and restarting the blood flow (210b);
        detecting a start Korotkoff sound and a stop Korotkoff sound of the restarted blood flow via the microphone array (210c); and
        estimating, via a trained deep learning model, a systolic BP and diastolic BP by processing a cuff pressure at the start Korotkoff sound and the stop Korotkoff sound sensed via a cuff pressure sensor (210d).

2. The method as claimed in claim 1, wherein the steady inflate pressure rate, and the steady deflate pressure rate is personalized for the subject by precomputing based on acquired profile information of the subject comprising gender, age and Body Mass Index (BMI).

3. A system (100) comprising:

an elastic cuff providing a sleeve fitting around an arm of a subject,
the elastic cuff (114) comprises an inner layer having an arrangement of the plurality of piezo electric sensors (302) spaced evenly into a plurality of quadrants, and a middle layer filled with a MR fluid (304), and an outer layer having an arrangement of a plurality of electromagnets (306) spaced evenly into the plurality of quadrants;
a triangular microphone array (308) placed above the outer layer, and
a cuff pressure sensor (310) to measure a cuff pressure.

4. The system as claimed in claim 3, wherein the elastic cuff is controlled by a controller (102) comprising a cuff inflation control system and a cuff deflation control system generating a plurality of control signals to the controller for actuating the plurality of electromagnets, acquiring audio signals captured by the triangular microphone array, and sensing pressure via the plurality of piezoelectric sensors.

5. The system as claimed in claim 4, wherein the controller (102) comprises:

   a memory (106) storing instructions;
   one or more Input/Output (I/O) interfaces (112); and
   one or more hardware processors (104) coupled to the memory (106) via the one or more I/O interfaces (112), wherein the one or more hardware processors (104) are configured by the instructions to:

   compute an initial pressure exerted across each of a plurality of quadrants of an elastic cuff (114) fitted on an arm of a subject, wherein the initial pressure exerted per quadrant is sensed by an inner layer of the elastic cuff (114) comprising the plurality of piezoelectric sensors (302) evenly placed across the plurality of quadrants, wherein the elastic cuff (114) comprises an outer layer of a plurality of electromagnets (306) placed evenly across the plurality of quadrants and a middle layer comprising a pre-compressed Magneto rheological (MR) fluid (304);
   compute an average pressure per quadrant by averaging the initial pressure sensed by each of the plurality of sensors (302) in associated quadrant;
   identify, a quadrant exerting a highest average pressure on the arm from among the plurality of quadrants, wherein the highest pressure is identified as an offset pressure value;
   actuate, via a Pulse Width Modulation (PWM) circuit, the plurality of electromagnets in remaining quadrants other than the quadrant of the highest average pressure to inflate the elastic cuff 114 to create a pressure equivalent to the offset value in the plurality of quadrants enabling a clean fitting of the elastic cuff 114 around the arm due to a viscosity change of the MR fluid under pressure; and
   initiate a Blood Pressure (BP) measurement cycle after detecting the pressure equivalent to the offset value in the plurality of quadrants, the BP measurement cycle comprising:

   actuating the plurality of electromagnets via the PWM circuit to inflate the elastic cuff (114) at a steady inflation rate by increasing the pressure at a steady rate until a blood flow in the arm stops, wherein the blood flow is sensed for a no-blood-flow-sound via the microphone array (308) placed within the elastic cuff;
   deactivating the plurality of electromagnets (306) to deflate the elastic cuff by releasing the pressure at a steady deflate pressure rate and restarting the blood flow;
   detecting a start Korotkoff sound and a stop Korotkoff sound of the restarted blood flow via the microphone array; and
   estimating via a trained Deep Learning model a systolic BP and diastolic BP by processing a cuff pressure sensed for the start Korotkoff sound and the stop Korotkoff sound by the cuff pressure sensor (310).

6. The system as claimed in claim 5, wherein the steady inflate pressure rate, and the steady deflate pressure rate is personalized for the subject by precomputing based on acquired profile information of the subject comprising gender, age and Body Mass Index (BMI).

7. One or more non-transitory machine-readable information storage mediums comprising one or more instructions which when executed by one or more hardware processors cause:

   computing an initial pressure exerted across each of a plurality of quadrants of an elastic cuff fitted on an arm of a subject, wherein the initial pressure exerted per quadrant is sensed by an inner layer of the elastic cuff further comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid;

computing an average pressure per quadrant by averaging the initial pressure sensed by each of a piezoelectric sensor among the plurality of sensors in associated quadrant;

identifying a quadrant exerting a highest average pressure on the arm from among the plurality of quadrants, wherein the highest pressure is identified as an offset pressure value;

actuating, via a Pulse Width Modulation (PWM) circuit, the plurality of electromagnets in remaining quadrants other than the quadrant of the highest average pressure to inflate the elastic cuff to create a pressure equivalent to the offset value in the plurality of quadrants enabling a clean fitting of the elastic cuff around the arm due to a viscosity change of the MR fluid under pressure; and

initiating a Blood Pressure (BP) measurement cycle after detecting the pressure equivalent to the offset value in the plurality of quadrants, the BP measurement cycle further comprising:

actuating the plurality of electromagnets via the PWM circuit to inflate the elastic cuff at a steady inflation rate by increasing the pressure at a steady rate until a blood flow in the arm stops, wherein the blood flow is sensed for a no-blood-flow-sound via a microphone array placed within the elastic cuff;

deactivating the plurality of electromagnets to deflate the elastic cuff by releasing the pressure at a steady deflate pressure rate and restarting the blood flow;

detecting a start Korotkoff sound and a stop Korotkoff sound of the restarted blood flow via the microphone array; and

estimating via a trained Deep Learning model a systolic BP and diastolic BP by processing a cuff pressure at the start Korotkoff sound and the stop Korotkoff sound sensed via a cuff pressure sensor.

8. The one or more non-transitory machine-readable information storage mediums of claim 7, wherein the one or more instructions which when executed by the one or more hardware processors further cause gender, age and Body Mass Index (BMI).

100

Controller 102

Processor(s) 104

Memory 106

Database 108

Modules 110

I/O Interface(s) 112

MR fluid filled-Elastic Cuff 114

FIG. 1A

EP 4 778 453 A1

100

Piezoelectric sensor array 302
(inner layer of cuff)

Pressure feedback
exerted on the limb

Controller 112
(Micro-processor)

Cuff fit
control
system

PWM
signal

Cuff pressure
Sensor 310
(attached to
cuff)

MR fluid filled
Cuff 114
(actuator)

Electromagnetic
layer 306
(magnetic filed)

Cuff
inflation
control
system

Microphone sensor
array 308
(attached to the
sleeve)

Blood flow
sound feedback

Korotkoff sound
feedback

Auscultation
based BP
prediction

Cuff pressure feedback

FIG. 1B

15

200

computing initial pressure exerted across each of a plurality of quadrants of an elastic cuff fitted on an arm of a subject, wherein the initial pressure exerted per quadrant is sensed by an inner layer of the elastic cuff comprising a plurality of piezoelectric sensors evenly placed across the plurality of quadrants, wherein the elastic cuff comprises an outer layer of a plurality of electromagnets placed evenly across the plurality of quadrants and a middle layer comprising a pre-compressed Magneto rheological (MR) fluid — 202

computing an average pressure per quadrant by averaging the initial pressure sensed by each of a piezoelectric sensor among the plurality of sensors in associated quadrant — 204

identifying a quadrant exerting a highest average pressure on the arm from among the plurality of quadrants, wherein the highest pressure is identified as an offset pressure value — 206

actuating a Pulse Width Modulation (PWM) circuit, the plurality of electromagnets in remaining quadrants other than the quadrant of the highest average pressure to inflate the cuff to create a pressure equivalent to the offset value in the plurality of quadrants enabling a clean fitting of the cuff around the arm due to a viscosity change of the MR fluid under pressure — 208

A

**FIG. 2A**

200

A

initiating a Blood Pressure (BP) measurement cycle after detecting the pressure equivalent to the offset value in the plurality of quadrants, the BP measurement cycle comprising:

actuating the plurality of electromagnets via the PWM circuit to inflate the cuff at a steady inflation rate by increasing the pressure at a steady rate until a blood flow in the arm stops, wherein the blood flow is sensed for a no-blood-flow-sound via a microphone array placed within the cuff (210a);

deactivating the plurality of electromagnets to deflate the cuff by releasing the pressure at a steady deflate pressure rate and restarting the blood flow (210b);

detecting a start Korotkoff sound and a stop Korotkoff sound of the restarted blood flow via the microphone array (210c); and

estimating via a trained Deep Learning model a systolic BP and diastolic BP by processing a cuff pressure sensed for the start Korotkoff sound and the stop Korotkoff sound (210d)

210

**FIG. 2B**

FIG. 3

FIG. 4

FIG. 5A

FIG. 5B

Europäisches
Patentamt
European
Patent Office
Office européen
des brevets

**EUROPEAN SEARCH REPORT**

Application Number

EP 25 20 3113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/126187 A1 (NEWSOUTH INNOVATIONS PTY LTD [AU]) 23 June 2022 (2022-06-23) * figures 4A-7 * * paragraphs [0010], [0017], [0050] - [0053], [0090] - [0098] * ----- | 1-8 | INV. A61B5/021 A61B5/022 A61B5/0225 |
| A | US 2012/253210 A1 (UESAKA CHISATO [JP] ET AL) 4 October 2012 (2012-10-04) * figure 12 * * paragraph [0095] * ----- | 1-8 | |
| A | US 2013/102910 A1 (PARK JONG-EUN [KR] ET AL) 25 April 2013 (2013-04-25) * figure 4 * * paragraphs [0078] - [0080] * ----- | 1-8 | |
| A | US 2009/036785 A1 (DANIELSSON PER [SE]) 5 February 2009 (2009-02-05) * figures 1-4 * * paragraphs [0018] - [0023] * ----- | 1-8 | |
| A | WO 2012/059907 A1 (GREISAS NISSIM [IL]; WACHTENBERG EYAL [IL]; GREISAS PEGGY [IL]) 10 May 2012 (2012-05-10) * figures 2,3 * * page 5, line 28 - page 6, line 7 * ----- | 1-8 | TECHNICAL FIELDS SEARCHED (IPC) A61B |
| A | US 6 251 061 B1 (HASTINGS ROGER N [US] ET AL) 26 June 2001 (2001-06-26) * figures 5A-5C * * column 5, line 63 - column 6, line 41 * ----- | 1-8 | |

The present search report has been drawn up for all claims

| Place of search | Date of completion of the search | Examiner |
|---|---|---|
| The Hague | 26 November 2025 | Oancea, A |

EPO FORM 1503 03.82 (P04C01)

## ANNEX TO THE EUROPEAN SEARCH REPORT
## ON EUROPEAN PATENT APPLICATION NO.

EP 25 20 3113

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2025

| Patent document cited in search report | | | Publication date | Patent family member(s) | | | Publication date |
|---|---|---|---|---|---|---|---|
| WO 2022126187 | A1 | | 23-06-2022 | NONE | | | |
| US 2012253210 | A1 | | 04-10-2012 | CN | 102715894 | A | 10-10-2012 |
| | | | | DE | 102012203049 | A1 | 04-10-2012 |
| | | | | JP | 2012200508 | A | 22-10-2012 |
| | | | | US | 2012253210 | A1 | 04-10-2012 |
| US 2013102910 | A1 | | 25-04-2013 | CN | 102970921 | A | 13-03-2013 |
| | | | | EP | 2591719 | A2 | 15-05-2013 |
| | | | | JP | 2013530769 | A | 01-08-2013 |
| | | | | KR | 101059109 | B1 | 25-08-2011 |
| | | | | US | 2013102910 | A1 | 25-04-2013 |
| | | | | WO | 2012005487 | A2 | 12-01-2012 |
| US 2009036785 | A1 | | 05-02-2009 | EP | 1983893 | A1 | 29-10-2008 |
| | | | | SE | 529044 | C2 | 17-04-2007 |
| | | | | US | 2009036785 | A1 | 05-02-2009 |
| | | | | WO | 2007089194 | A1 | 09-08-2007 |
| WO 2012059907 | A1 | | 10-05-2012 | NONE | | | |
| US 6251061 | B1 | | 26-06-2001 | US | 6251061 | B1 | 26-06-2001 |
| | | | | WO | 0013722 | A1 | 16-03-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202521004029

**Non-patent literature cited in the description**

- **BEEVERS, G.** ; **LIP, G.Y.** ; **O'BRIEN, E.** Blood pressure measurement: Part ii-conventional sphygmomanometry: Technique of auscultatory blood pressure measurement. *Bmj*, 2001, vol. 322 (7293), 1043-1047

- **PAN, F.** ; **HE, P.** ; **CHEN, F.** ; **ZHANG, J.** ; **WANG, H.** ; **ZHENG, D.** A novel deep learning based automatic auscultatory method to measure blood pressure. *International journal of medical informatics*, 2019, vol. 128, 71-78